# EUROPEAN PATENT SPECIFICATION

(11) **EP 2 799 100 B1**
(45) Date of publication and mention of the grant of the patent: **07.11.2018**
(21) Application number: 14166596.8
(22) Date of filing: 30.04.2014
(51) Int. Cl.: A61M 5/20, A61M 5/315

(54) **Key for securing components of a drug delivery system during assembly and/or transport and methods of using same**
Schlüssel zur Sicherung von Komponenten eines Arzneimittelabgabesystems während der Montage/des Transports und Verfahren zur Verwendung davon
Clé pour fixer des composants d'un système d'administration de médicament pendant l'assemblage et/ou le transport et méthodes d'utilisation associées

(30) Priority: 30.04.2013 US 201313874121
(43) Date of publication of application: 05.11.2014
(73) Proprietor: Medimop Medical Projects Ltd., 43665 Ra'anana (IL)
(72) Inventor: Degitiar, Boris, 71721 Modiin (IL); Bar-El, Yossi, 71947 Beit Arye (IL); Filman, Reuven Y., 42560 Netanya (IL)
(74) Representative: Patentanwälte Vollmann & Hemmer

(56) References cited:
- EP-A1- 1 530 979
- WO-A1-2004/105841
- US-A- 4 698 055
- US-A1- 2013 085 457

## Description

This application refers to US 2009/0093792 A1 and to the U.S. Patent Application 13/874,085 entitled DISENGAGEMENT RESISTANT TELESCOPING ASSEMBLY AND UNIDIRECTIONAL METHOD OF ASSEMBLY FOR SUCH.

The present invention, in some embodiments thereof, relates to drug delivery systems and, more particularly, but not exclusively, to apparatuses and methods for securing components of a drug delivery system during assembly and/or transport.

Failure to use a delivery device or system, such as an insulin pen or auto-injector, correctly could result in a life or death emergency, or impact a patient's or caregiver's ability to manage a medical condition effectively. For the pharmaceutical manufacturer, such a failure could result in a massive backlash that may cause loss of market share, costly product recalls or worse.

The primary goal of any drug delivery system is to ensure that a patient receives a proper dose of a prescribed drug. In years past, if a device failed or was used incorrectly, patient or caregiver error was most often the culprit. While providing detailed instructions is important for any pharmaceutical manufacturer, failure to follow directions is no longer a viable excuse when a patient or caregiver is unable to operate a device or delivery system successfully.

Effective drug therapy and treatment typically involves more than simply having an effective molecule. Rather, it is the combination of a safe drug within a suitable container and/or delivery system.

Historically, pharmaceutical manufacturers have focused, and rightly so, on the efficacy and safety of the drug product. However, if the drug is to achieve its therapeutic objective, then its primary container and delivery system must be both compatible with the drug and stable over time, as well as foster adherence from the patient. A drug can only truly have the desired patient benefit if it is taken as prescribed, delivered effectively (often by a patient or caregiver), and maintains performance over time.

Today's injectable therapies can take many forms. Liquid drugs may use a traditional syringe and vial; a prefilled syringe; or a delivery system such as an auto-injector, pen device or patch injector. Lyophilized drug products (requiring reconstitution with water for injection) may use a kit containing a transfer device, syringe or needle, and containers of the drug and water.

The container format itself also should be considered. Vials may be used for initial use, but a syringe or cartridge system may provide the best solution for the patient when the system reaches the market. Once the primary container has been selected, efforts must be made to ensure that it works with the delivery system. Dimensional tolerances and functionality should be tested to ensure proper activation and gliding forces.

Recognizing how the patient or caregiver interacts with the delivery system is essential to ensuring success in the market. Even the most innovative drug can provide the appropriate therapeutic benefit to the patient only if it can be delivered effectively and the patient adheres to the treatment regimen. Patients or caregivers may choose one product over another based on dose frequency, pain associated with dosing, or ease of use or mobility of the delivery system. Simply put, packaging can differentiate a product's market acceptance.

One frequent ease of use issue that is encountered by users of drug delivery systems, for example patch injectors like the SmartDose® Electronic Patch Injector System offered by Medimop Medical Projects Ltd., a subsidiary of West Pharmaceutical Services, Inc., is movement of the operative parts during transport. For example, vibrations during transport may cause movements of screws causing a telescoping assembly (TSA) of the delivery system to extend. As result, when a cartridge containing the unintentionally extended telescoping assembly is inserted by the user, it may be difficult to close the door of the delivery system. Some users may interpret this as a malfunction and elect not use the unit.

US 4 698 055 A a hypodermic syringe in which a first medicament is stored in an elongated barrel, adjacent its distal end, while a second medicament is stored in an exterior compartment between the distal and proximal ends in communication with the barrel. A plunger having a piston head with spaced apart sealing rings is located in the syringe barrel with the opposing sealing rings occluding the exterior compartment so as to isolate the medicaments from each other. The piston is normally locked in this position. In use, the piston is moved adjacent the proximal end of the barrel to allow the medicament in the exterior compartment to intermix with the first medicament before dispensing.

International Patent Application Publication No. WO/2011/090956 describes a cartridge interface assembly characterized by a driving plunger including an outer shaft, and a driver including an inner shaft movable telescopically with respect to the outer shaft, wherein rotation of the driver causes the driving plunger to advance in a direction away from the driver. When the cartridge interface assembly is inserted in a cartridge in which a plunger is slidingly disposed, rotation of the driver causes the driving plunger to advance distally in the cartridge until abutting against the plunger.

The shafts may tend to unscrew during transportation and handling before assembly, with the result that the position, which is the desirable position for assembly with the cartridge plunger, is not maintained. On the other hand, if the driver is tightened too much against the body of assembly in an effort to maintain the closed position of, this can increase the torque used by the motor to overcome the tight connection in order to start turning the driver, thereby overburdening the motor. To solve this double problem (possible opening of telescoping shafts or the driver being tightened too much), a locking assembly is provided

An aspect of the invention relates to an assembly key used for securing a telescoping assembly of a drug delivery system during transport. In some embodiments of the invention, the drug delivery system is a patch injector.

The assembly key is provided with a locking bit which is adapted to interface with at least one component of the telescoping assembly. The locking bit may be inserted into a key slot located on a pushing nut screw of the telescoping assembly. The locking bit first passes through a bit aperture of a cartridge gear of the telescoping assembly, thereby linking the cartridge gear and pushing nut screw together, but also substantially or entirely preventing the turning of the telescoping assembly, for example as a result of vibrations during transport.

Optionally, the assembly key may be removably attached to the cartridge gear. At least one clip is provided to the assembly key which snaps into at least one counterpart clip aperture located on the cartridge gear.

Optionally, the pushing nut screw may be at least partially deformed in order to retain a mid screw located within the pushing nut screw. In an embodiment of the invention, retention of the mid screw substantially prevents rotation of the telescoping assembly, and thereby effectuates substantial stoppage of unintended extension of the telescoping assembly.
Another aspect of the invention relates to a method for assembling a telescoping assembly and securing it for transport. In an embodiment of the invention, a mid screw of the telescoping assembly is threaded onto on internal screw of the telescoping assembly. A cartridge gear of the telescoping assembly is applied onto the internal screw from the same end of the internal screw that the mid screw was threaded, in an embodiment of the invention. Optionally, the three assembled components (the mid screw, the internal screw and the cartridge gear) are threaded into a pushing nut screw of the telescoping assembly, which is already attached to a pushing nut cover of the telescoping assembly.

Optionally, the three assembled components (the mid screw, the internal screw and the cartridge gear) may be screwed into the pushing nut screw so that a bit aperture located on the cartridge gear is aligned with a key slot on the pushing nut screw.

An assembly key is installed onto the completed telescoping assembly by inserting a locking bit located on the assembly key into the bit aperture and further, into the key slot.

Optionally, at least one clip provided to the assembly key may be inserted through at least one counterpart clip aperture located on the cartridge gear to removably attach the assembly key to the telescoping assembly.

The locking bit being inserted into the locking bit aperture and into the key slot substantially prevents rotation of the pushing nut screw and/or the unintended extension of telescoping assembly as a result of vibrations during transport.

Optionally, the assembly key may be removed from the telescoping assembly after transport to enable normal operation of the telescoping assembly and the drug delivery system.

Optionally, the assembly key may be economically manufactured and/or may be constructed of inexpensive materials. Optionally, the assembly key may be disposable and may be disposed of after use.

Optionally, the assembly key may be removed from the telescoping assembly after transport to enable normal operation of the telescoping assembly and the drug delivery system.

Optionally, the pushing nut screw may be at least partially deformed near or at the end opposite the pushing nut cover to create a stop for the mid screw, creating an additional means for securing the telescoping assembly during transport. Optionally, the stop is created by driving a shot pin into the pushing nut screw 104, possibly heated or ultrasonic.

Optionally, the assembly key may secure a telescoping assembly in a desired configuration and/or may assist assembly of the telescoping assembly into a medicine dispensing device. For example, a telescoping assembly may be delivered to a drug supplier for assembly into a syringe containing a drug. The drug supplier may need to orient the telescoping assembly and/or insert a portion of the assembly into a syringe and/or connect the telescoping assembly to a plunger inside the syringe. An assembly key may optionally have markings and/or a geometry to assist orientation of the telescoping assembly. The assembly key may optionally secure the telescoping assembly in the proper configuration for assembly to the syringe. The assembly key may optionally provide a gripping surface for attaching the telescoping assembly to the plunger of the syringe. Optionally, the plunger is considered to be a component of the telescoping assembly.

Optionally, the assembly key may secure a telescoping assembly in a desired configuration for deployment by an end user. An end user may optionally insert a cartridge into a drug delivery device (for example an infuser and/or a patch injector) and/or administer the drug. An assembly key may optionally include markings and/or a geometry to assist orientation of the telescoping assembly. The assembly key may optionally secure the telescoping assembly in the proper configuration for insertion into a delivery device and/or drug delivery. In some embodiments, the assembly key may be removed before the beginning of drug delivery. The assembly key may optionally include features that assist simple, intuitive removal of the key before use.

There is provided in accordance with an aspect of the invention, a method of securing an assembly of a drug delivery system according to claim 1.

Optionally, the method may further comprise employing the assembly key to facilitate assembly of the assembly during manufacture.

Optionally, the transporting may be to an end user.

Optionally, the method may further comprise removing the assembly key after transporting.

Optionally, the method may further comprise disposing of the assembly key after removing.

Optionally, the removing may be performed by the end user.

The securing includes preventing relative rotation of at least two components of the assembly.

The installing the assembly key includes inserting the locking element of the assembly key through a bit aperture of a first component of the assembly and into a slot, aligned with the bit aperture, of a second component of the assembly, thereby preventing relative movement of the first component and the second component.

Optionally, installing the assembly key may include inserting the locking element of the assembly key through a key aperture of a first component of the assembly and into a key slot, aligned with the key aperture, of a second component of the assembly, thereby preventing relative movement of the first component and the second component.

Optionally, installing may comprise removably inserting at least one clip into a first component of the assembly.

Optionally, the at least one clip may be installed through the top of the first component.

Optionally, the at least one clip may be installed on the outside of the first component.

Optionally, a driver component may be the first component and an actuating component may be the second component.

Optionally, the driver component may include a gear.

Optionally, the actuating component may include a pushing nut cover configured for pushing a plunger of a syringe.

Optionally, the method may further comprise providing the assembly key with a distinguishing feature to make the assembly key easier to identify for removing.

Optionally, the method may further comprise using the assembly key as a mechanical linkage between the assembly and an assembly machine during manufacture of the assembly key prior to transporting.

Optionally, the method may further comprise using the assembly key to sense the orientation of the assembly during manufacturing.

Optionally, the method may further comprise using the assembly key to screw the assembly into a plunger of the drug delivery system.

The step of installing includes passing a locking bit of the assembly key through a bit aperture in a first of the at least two components and into a key slot of a second of the at least two components.

There is provided, in accordance with an aspect of the invention, a system for preventing unintended linear extension of an assembly of a drug delivery system during transport according to claim 5.

The at least the first component and the second component are interconnected by a screw thread, wherein relative rotation of the first and second component causes extension of the assembly.

The assembly key includes at least one locking element configured to interplay with at least the first component and the second component such that the two components are prevented from rotating with respect to each other, thereby preventing linear extension of the assembly.

Optionally, the first component of the assembly may include an actuating component for pushing a plunger of the drug delivery system.

Optionally, the first component may be provided with a key slot adapted as a counterpart to the locking element.

Optionally, the second component may include a driver component.

Optionally, the second component may include a gear.

Optionally, the second component may be provided with a key aperture adapted to the locking element therethrough.

Optionally, the key aperture on the second component may align with a key slot on the first component to pass the locking element through the first and second components together.

Optionally, the assembly key may further comprise at least one clip for removably attaching the assembly key to at least one component of the assembly.

Optionally, the system may further comprise at least one clip aperture provided to the at least one component of the assembly configured as a counterpart to the at least one clip.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below.. In addition, the materials, methods, and examples are illustrative only and are not intended to be necessarily limiting.

Some embodiments of the invention are herein described, by way of example only, with reference to the accompanying drawings. With specific reference now to the drawings in detail, it is stressed that the particulars shown are by way of example, are not to scale, and are for purposes of illustrative discussion of embodiments of the invention. In this regard, the description taken with the drawings makes apparent to those skilled in the art how embodiments of the invention may be practiced.

In the drawings:
FIG. 1 is a block diagram illustrating a system for preventing unintended extension of a telescoping assembly of a drug delivery system, in accordance with an exemplary embodiment of the invention;
FIG. 2 is an exploded perspective view of a telescoping assembly of a drug delivery system, in accordance with an exemplary embodiment of the invention;
FIG. 3 is a perspective view of a closed telescoping assembly of a drug delivery system with an assembly key, in accordance with an exemplary embodiment of the invention;
FIG. 4 is a perspective, cross-sectional view of the closed telescoping assembly with assembly key of FIG. 3, in accordance with an exemplary embodiment of the invention;
FIG. 5 is a perspective view of the closed telescoping assembly of FIG. 3 showing the top of the cartridge gear with the assembly key removed, in accordance with an exemplary embodiment of the invention;
FIGS. 6A-6C are perspective views of the assembly key, in accordance with an exemplary embodiment of the invention;
FIG. 7A is a perspective view of a telescoping assembly showing a key slot in a component of the assembly which acts as a counterpart to the assembly key, in accordance with an exemplary embodiment of the invention;
FIG. 7B is a close-up, perspective view of the assembly key engaged with the key slot, in accordance with an exemplary embodiment of the invention;
FIG. 8 is a flowchart of a method for assembling a telescoping assembly of a drug delivery system while also securing it for transport, in accordance with an exemplary embodiment of the invention;
FIG. 9 is a flowchart of an alternative method for assembling a telescoping assembly of a drug delivery system while also securing it for transport, in accordance with an exemplary embodiment of the invention;
FIG. 10 is a flowchart of a method for using an assembly key to secure a telescoping assembly of a drug delivery system, in accordance with an exemplary embodiment of the invention;
FIG. 11A is a perspective view of an alternative telescoping assembly showing a key slot in a component of the assembly which acts as a counterpart to an alternative assembly key, in accordance with an exemplary embodiment of the invention;
FIG. 11B is a perspective view of the assembled, closed alternative telescoping assembly of FIG. 11A, in accordance with an exemplary embodiment of the invention;
FIG. 11C is a cross-sectional view of the alternative telescoping assembly with assembly key of FIG. 11B, in accordance with an exemplary embodiment of the invention; and,
FIGS. 12A-12C are perspective views of the alternative assembly key, in accordance with an exemplary embodiment of the invention.

The present invention, in some embodiments thereof, relates to drug delivery systems and, more particularly, but not exclusively, to apparatuses and methods for securing components of a drug delivery system during transport.

Before explaining at least one embodiment of the invention in detail, it is to be understood that the invention is not necessarily limited in its application to the details of construction and the arrangement of the components and/or methods set forth in the following description and/or illustrated in the drawings and/or the exemplary embodiments. The invention is capable of other embodiments or of being practiced or carried out in various ways within the scope of the claims.

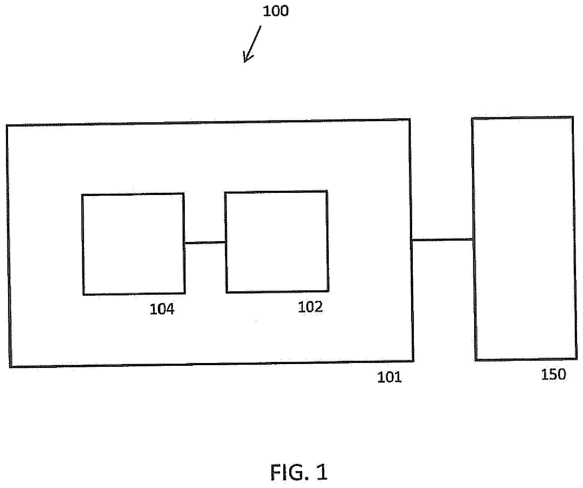

Referring now to the drawings, FIG. 1 is a block diagram illustrating a system 100 for preventing unintended movement of an assembly 101 of a drug delivery system, in accordance with an exemplary embodiment of the invention. In an embodiment of the invention, the system is comprised of telescoping assembly and an assembly key 150. In an embodiment of the invention, the assembly key 150 is a separate component from the assembly, provided with latches and/or clips to removably attach it to at least one component of the assembly and which substantially retains the assembly 101 in a set configuration by preventing the linear movement of at least a first component 102 of the telescoping assembly 101 with respect to at least a second component 104 of the assembly 101. In an embodiment of the invention, the set configuration is a closed configuration.

In some embodiments of the invention, extension of the telescoping assembly 101 is achieved by rotation of at least one of the components of the assembly 101. For example, the assembly key 150 substantially prevents rotation of the assembly 101 by interlocking at least two components of the assembly 101 to prevent their rotation with respect to each other.

In some embodiments of the invention, the drug delivery system is a patch injector system. In some embodiments of the invention, the telescoping assembly 101 is an operative component of a cartridge of a patch injector system, wherein the disposable and/or interchangeable cartridge contains a pre-measured dose of a drug to be administered to a patient using the drug delivery system.

It should be understood that in order to utilize a smaller, more economical motor, the force used to screw the telescoping assembly 101 in order to extend and retract it may optionally be minimized with low friction threading. This low friction threading allows for easier movement using the motor, but it is also what allows for easy unintended movement of the assembly 101 as a result of vibrations, for example during transport.

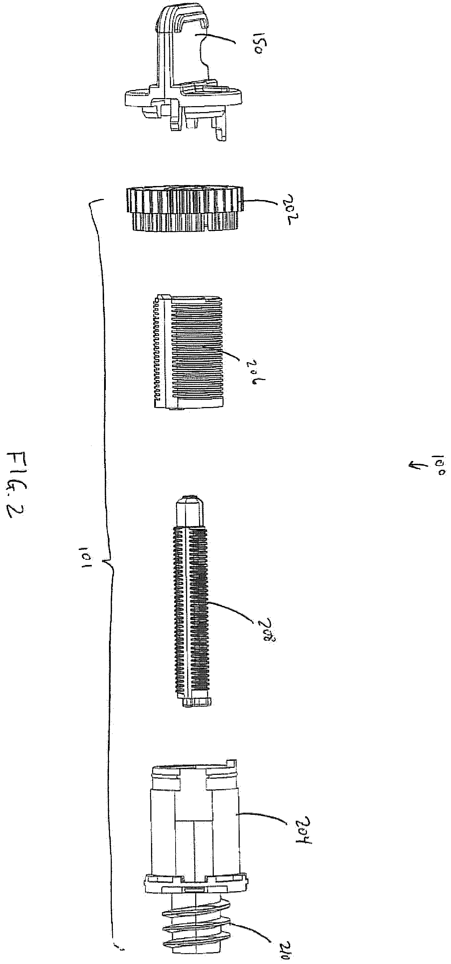

FIG. 2 illustrates an exploded perspective view of a telescoping assembly 101 of a drug delivery system, in accordance with an exemplary embodiment of the invention. In an embodiment of the invention, telescoping assembly 101 is comprised of at least one driver component, for example a cartridge gear 202, and/or at least one external screw, for example a pushing nut screw 204, and/or at least one mid screw 206, and/or an internal screw 208 and/or a actuating component, for example a pushing nut cover 210 (shown in FIG. 2 pre-attached to pushing nut screw 204) for pushing a syringe plunger. Telescoping assembly 101 includes three elongated threaded elements (pushing nut screw 204, mid screw 206 and internal screw 208) which move longitudinally with respect to one another to telescope (extend or close) the assembly, in an embodiment of the invention. The elongated elements may be interconnected by screw threads which drive the longitudinal movement when the elongated elements are rotated relative to one another. Alternatively or additionally, a telescoping assembly may include two, four or more elongated elements that may move longitudinally with respect to each other and/or may be interconnected by screw threads and/or may be driven by relative rotation.

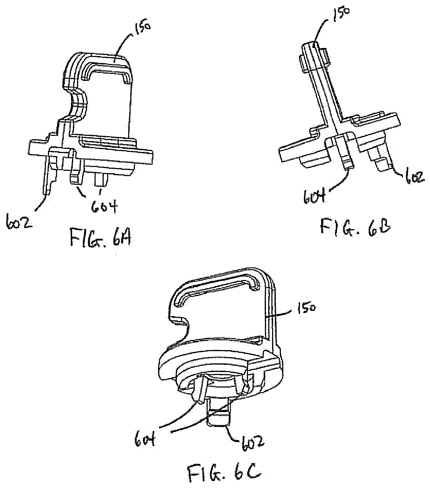

An assembly key 150 is provided to the telescoping assembly 101 to secure the telescoping assembly 101 for transport and to optionally assist with assembly of the telescoping assembly 101 into a syringe and/or a plunger of the syringe. In some exemplary embodiments of the invention, the telescoping assembly 101 is configured to interplay with assembly key 150 to secure the telescoping assembly 101 for transport and/or assembly. For example, the telescoping assembly 101 is configured to be secured by inserting a locking element, for example, locking bit 602, shown and described in more detail with respect to FIGS. 6A-7B, of the assembly key 150 into at least one component of the telescoping assembly 101 thereby locking it to prevent unintended extension of the assembly 101, in an exemplary embodiment of the invention. In some embodiments of the invention, the at least one component of the assembly 101 into which the locking bit 602 is inserted is pushing nut screw 204.

The pushing nut cover 210 may be attached to a plunger or stopper (not shown) in the cartridge of the patch injector. The plunger may provide a fluid proof seal against the liquid in the cartridge and which pushes the fluid out of the cartridge and into the patient when the telescoping assembly 101 extends and activates/instigates the pushing of the pushing nut cover 210.

The at least one component may be manufactured from a medical grade polymer, for example Polybutylene Terephthalates (PBTs), Ticona Celanex 2402MT, Ticona Celanex 2405MT, Delrin 500AL and Delrin 500P. In some embodiments of the invention, at least one component is manufactured from a plurality of materials, for example the gears are Ticona Celanex 2402MT but the threads are Ticona Celanex 2405MT. The cartridge gear may be approximately 12mm in diameter. In some embodiments of the invention, the extension achieved by the telescoping assembly is approximately 11 mm (for example, the 11 mm is a total amount of extension of a plurality of individual components which extend and contribute to the total), although it should be understood that virtually any length of extension could be achieved by any number of components operatively connected to one another. In some embodiments of the invention, the assembly key diameter approximately matches that of the cartridge gear.

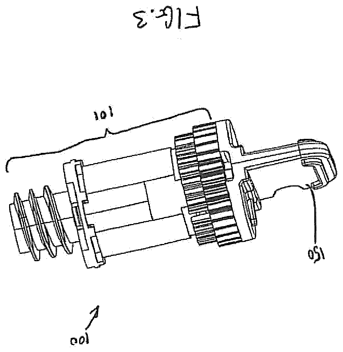

FIG. 3 is a perspective view of a telescoping assembly 101 with an assembly key 150 of a drug delivery system that is closed and locked by assembly key 150, in a typical secured configuration for transport and/or assembly and/or use in accordance with an exemplary embodiment of the invention.

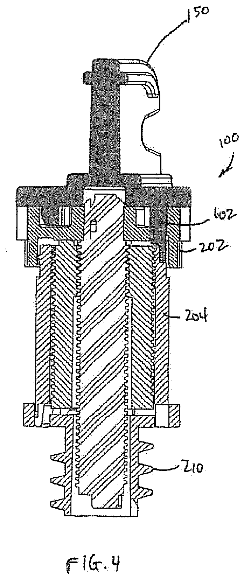

FIG. 4 is a perspective, cross-sectional view of the closed telescoping assembly 101 of FIG. 3, in accordance with an exemplary embodiment of the invention. In the example of FIG. 3, assembly key 150 is locked to cartridge gear and/or locking bit 602 of the assembly key 150 is shown interfacing with the pushing nut screw 204. In an embodiment of the invention, assembly key prevents the turning of the pushing nut screw 204 with respect to cartridge gear 202, thereby preventing substantially or entirely the unintended extension of the telescoping assembly. A handle on the proximal end of assembly key 150 may provide a gripping point and/or locking bit 602 may provide a torque point for twisting pushing nut screw 204 and/or pushing nut cover 210 For example, nut cover 210 may be inserted into a syringe and assembly key 150 may be used to twist pushing nut cover 210, screwing it into the plunger.

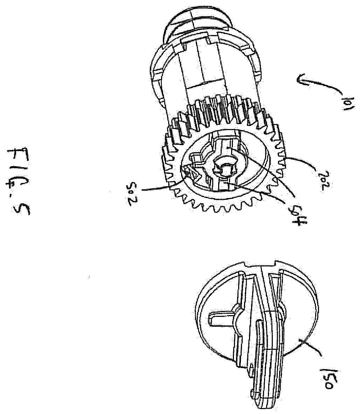

FIG. 5 is a perspective view of the closed telescoping assembly 101 of FIG. 3 showing the top of the cartridge gear 202 with the assembly key 150 removed, in accordance with an exemplary embodiment of the invention. In some embodiments of the invention, locking bit 602 is inserted through a bit aperture 502 in the cartridge gear 202 before it engages with a slot in the pushing nut screw 204. The assembly key 150 is provided with at least one clip 604, shown and described in more detail in FIGS. 6A-6C, which is removably attached to telescoping assembly 101 by inserting the at least one clip 604 through at least one counterpart clip aperture 504 located on the cartridge gear 202, in some embodiments of the invention. Additionally or alternatively, clip 604 may serve as a locking element, for example locking assembly key 150 to cartridge gear 202 and/or pushing nut screw 204. Additional details are shown in FIG. 4, wherein the at least one clip 604 is shown passing through a clip aperture 504 and through cartridge gear 202, in an embodiment of the invention.

FIGS. 6A-6C are perspective views of the assembly key 150, in accordance with an exemplary embodiment of the invention. Shown in more detail in the various views are the locking bit 602 and the at least one clip 604.

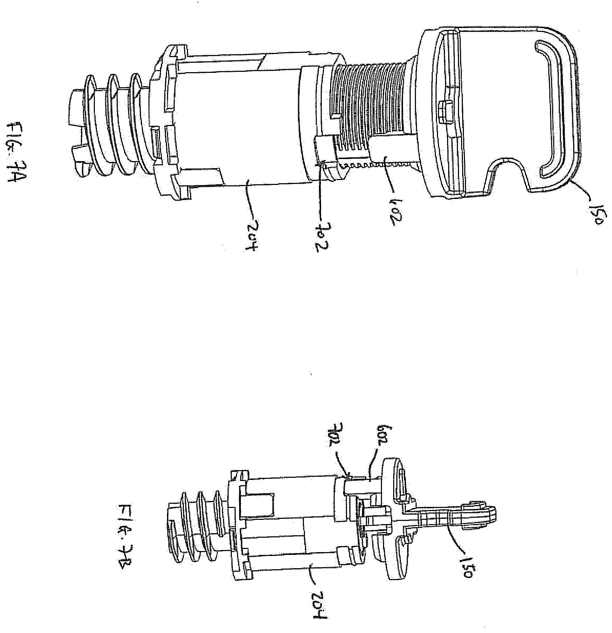

FIG. 7A is a perspective view of a telescoping assembly 101 showing a key slot 702 in a component of the assembly 101 which acts as a counterpart to the assembly key 150, in accordance with an exemplary embodiment of the invention. In some embodiments of the invention, the key slot 702 is located on the assembly key 150 key side of the pushing nut screw 204. In some embodiments of the invention, the key slot 702 aligns with the bit aperture 502 of the cartridge gear 202 (not shown in FIGS. 7A-7B, but shown in FIG. 5) whereby the locking bit 602 can be inserted through the bit aperture 502 and into the key slot 702.

FIG. 7B is a close-up, perspective view of the locking bit 602 of the assembly key 150 engaged with the key slot 702, in accordance with an exemplary embodiment of the invention. FIG. 7B does not show the cartridge gear 202 to make this configuration more visible, which in an embodiment of the invention would nominally surround the viewable portion of the pushing nut screw 204 and locking bit 602 in this Figure.

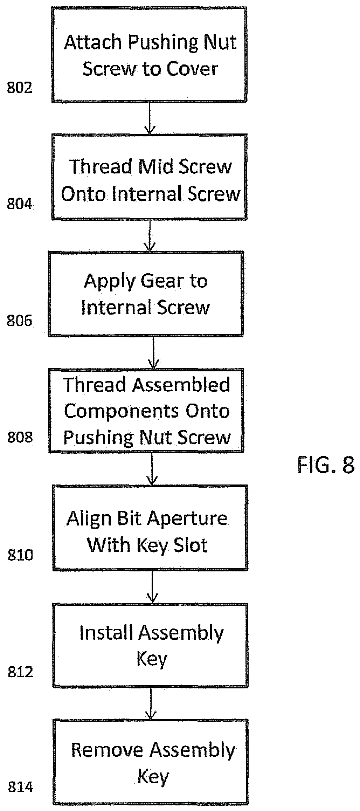

FIG. 8 is a flowchart of a method for configuring a telescoping assembly 101 of a drug delivery system and/or securing it for assembly and/or transport and/or deployment, in accordance with an exemplary embodiment of the invention. In an embodiment of the invention, the mid screw 206 is threaded (804) onto the internal screw 208 so that the threads on the exterior circumference of the mid screw are positioned substantially towards the pushing nut screw cover 210. The cartridge gear 202 is applied (806) onto the internal screw 208 at the end opposite the pushing nut cover 210, in an embodiment of the invention. In an embodiment of the invention, the three components 202, 206, 208 are threaded (808) into the pushing nut screw 204 by engaging the threads on the exterior circumference of the mid screw 206 with threads located on the inner circumference of the pushing nut screw 204, which is already attached (802) to the pushing nut cover 210. In an embodiment of the invention, the three components are screwed into the pushing nut screw 204 so that the bit aperture 502 located on the cartridge gear 202 is aligned (810) with the key slot 702 on the pushing nut screw 204.

The assembly key 150 is installed (812) onto the telescoping assembly 101 by inserting the locking bit 602 into the bit aperture 502 and further, into the key slot 702. Additionally and concurrently, the at least one clip 604 is inserted through the at least one counterpart clip aperture 504 located on the cartridge gear 202 to removably attach the assembly key 150 to the telescoping assembly 101. In an embodiment of the invention, the locking bit 602 inserted into the locking bit aperture 502 and into the key slot 702 substantially prevents rotation of the pushing nut screw 204 with respect to the cartridge gear 202. Assembly key 150 may optionally supply a way of aligning and/or gripping pushing nut screw 204 and/or cartridge gear 202, Assembly key 150 may optionally prevent the unintended extension and/or closing of telescoping assembly 101.

In an embodiment of the invention, the assembly key 150 is removed (814) from the telescoping assembly 101 after transport to enable normal operation of the telescoping assembly 101 and the drug delivery system. Removal (814) of assembly key 150 may be by simple pulling away from the telescoping assembly. Simple removal (814) of assembly key may contribute to the ease of use of the device.

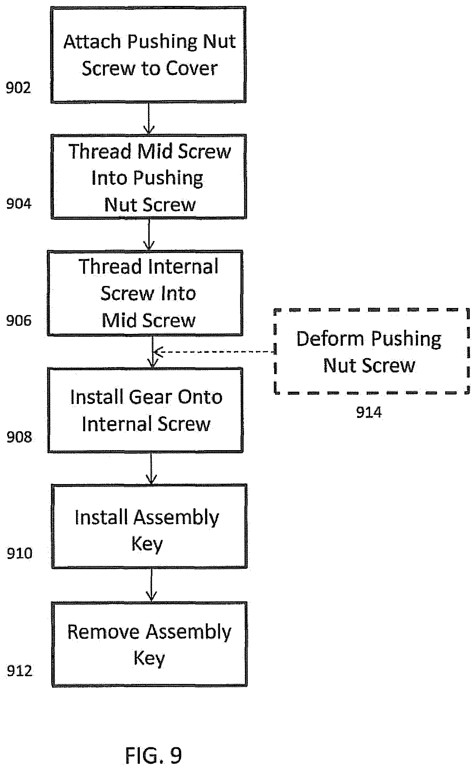

FIG. 9 is a flowchart of an alternative method for configuring a telescoping assembly 101 of a drug delivery system while also securing it for transport, in accordance with an exemplary embodiment of the invention. In an embodiment of the invention, pushing nut screw 204 is attached (902) to the pushing nut cover 210. The mid screw 206 is threaded (904) into the pushing nut screw 204 by engaging the threads on the exterior circumference of the mid screw 206 with threads on the interior circumference of the pushing nut screw 204. In an embodiment of the invention, the internal screw 208 is threaded (906) into the mid screw 206. The internal screw 208 is optionally over rotated to make installation of the cartridge gear 202 easier. In an embodiment of the invention, the cartridge gear 202 is installed (908) onto the inner screw aligning the bit aperture 502 of the cartridge gear 202 with the key slot 702 of the pushing nut screw 204 and, if the inner screw 208 was over rotated, the inner screw 208 is returned to a stop position.

In some embodiments of the invention, the pushing nut screw 204 is at least partially deformed (914) near or at the end opposite the pushing nut cover 210 to create (912) a stop for the mid screw 206. Optionally, the stop is created by driving a shot pin into the pushing nut screw 204, possibly heated or ultrasonic.

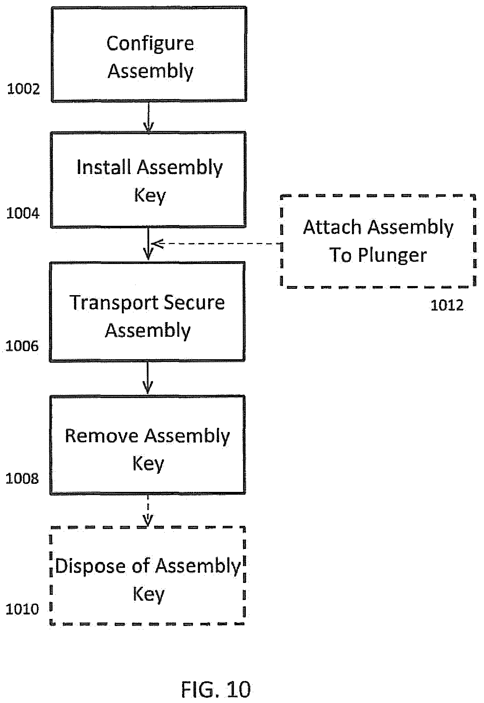

FIG. 10 is a flowchart illustration of a method for assembling and using a drug delivery system, in accordance with an exemplary embodiment of the invention. In an embodiment of the invention, the telescoping assembly is configured (1002) for use, for example as described in FIGS. 8 and/or 9. In an embodiment of the invention, the assembly key 150 is installed (1004) on the telescoping assembly 101, securing the assembly 101. For example, securing may include removably attaching the assembly key 150 to at least one component of the telescoping assembly 101. The telescoping assembly is now secured, preventing unintended extension, for assembly and/or transport and/or deployment.

In some embodiments, after telescoping assembly 101 is configured (1002) and secured (1004), it may be placed into a drug delivery system. Optionally, assembly key 150 assists attachment (1012) of the telescoping assembly 150 into the syringe. For example, pushing nut cover 210 may be attached to a plunger of a syringe. Assembly key, supply a convenient gripping point and/or alignment indication for a person and/or a machine to hold pushing nut cover 210 and/or to insert it into the syringe and/or to align threads of pushing nut cover 210 to threads in the plunger and/or to twist pushing nut cover 210 to screw it into the plunger.

In some embodiments, the syringe and/or the telescoping assembly 101 may be transported (1006) to the end user. The assembly key 150 is left on the telescoping assembly 101 during transport (1006). The assembly key 150 prevents the telescoping assembly 101 from moving out of its proper configuration for example due to vibrations during transport (1006).

In some embodiments, once the syringe and/or the telescoping assembly 101 reach the end user, the assembly key may be removed (1008) and/or the syringe and/or the telescoping assembly 101 may be deployed. For example deployment may include inserting the syringe and/or assembly 101 into a delivery device (for example a patch injector). Transporting (1006) the telescoping assembly 101 with the assembly key 150 may help prevent the telescoping assembly 101 from extending during transport (1006). Unwanted extension of the telescoping assembly beyond its closed configuration might, for example, make it difficult to fit the assembly 101 and/or the syringe/cartridge into the injector.

The delivery device may optionally restrain the syringe and/or the plunger and/or pushing nut cover 210 from rotating while rotating cartridge gear 202 causing the telescoping assembly 101 to extend, pushing the plunger into the syringe and/or delivering the drug. In some cases, transporting (1006) telescoping assembly 101 with the assembly key 150 attached (1004) may prevent telescoping assembly 101 from closing up too much during transport. Over closing of the telescoping assembly 101 may for example cause thread lock within the telescoping assembly 101 which could cause failure of the delivery system.

In an embodiment of the invention, the assembly key 150 and/or the entire drug delivery system is disposed of (1010). For example, the delivery key 150 and/or the telescoping assembly and/or the delivery device may be made of safe materials that may be disposed of (1010) in municipal garbage.

With respect to the assemblies and methods described herein it should be understood that, according to the invention, the assembly 101, which is secured for transport as described herein, remains in a closed configuration and does not unintentionally linearly extend as a result of vibrations during transport. In an embodiment of the invention, "transport" is to be understood as moving from the manufacturer, through the distribution chain, and ultimately to the consumer. In some embodiments, "transport" is a sub-set of the chain described above. For example, as applied to transport on a truck from a port of entry to a distribution center. In an embodiment of the invention, such as any of those described herein, the assembly key provides sufficient locking force to ensure compliance under the ASTM D4169 performance testing of shipping containers and systems standards for combined air and rail transport. In some embodiments of the invention, the assembly key prevents unintended linear extension of the assembly while being subjected to extended vibrations up to 300 Hz. Optionally, extended vibration time is for hours, days or even weeks, for example in the case of cargo shipping overseas. In some embodiments of the invention, the assembly key prevents unintended linear extension of the assembly while being subjected to shocks up to 300 m/s2.

The secured assembly 101 is placed into a drug delivery system, for example a patch injector like the SmartDose® Electronic Patch Injector System offered by Medimop Medical Projects Ltd., a subsidiary of West Pharmaceutical Services, Inc. after the removal of the assembly key 150, in accordance with an exemplary embodiment of the invention.

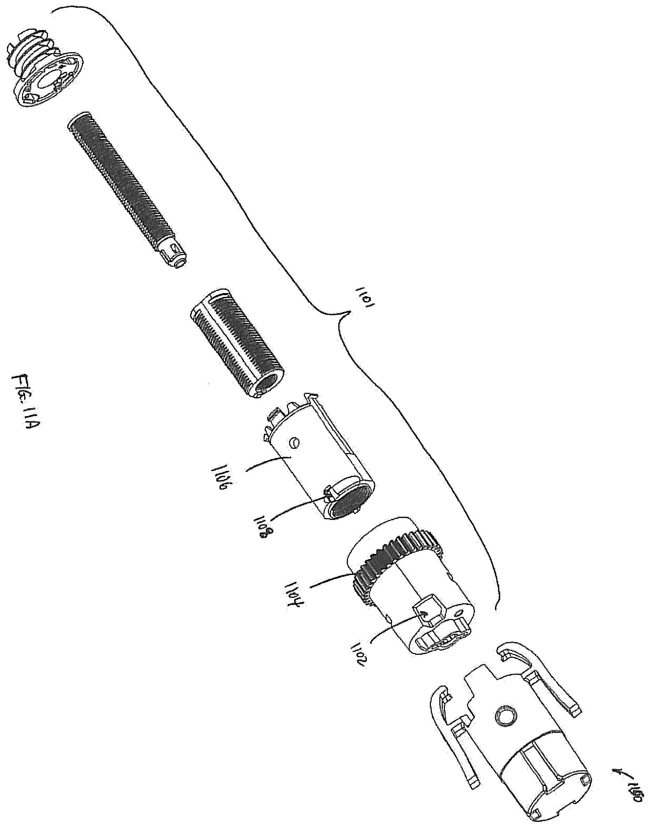

FIG. 11A is a perspective view of an alternative telescoping assembly 1101 showing at least one bit aperture 1102 in at least one component of the assembly which acts as a counterpart to at least one locking bit 1152 of an assembly key 1150, in accordance with an exemplary embodiment of the invention. In some embodiments of the invention, the bit aperture 1102 is located on the assembly key 150 key side of the cartridge gear 1104.

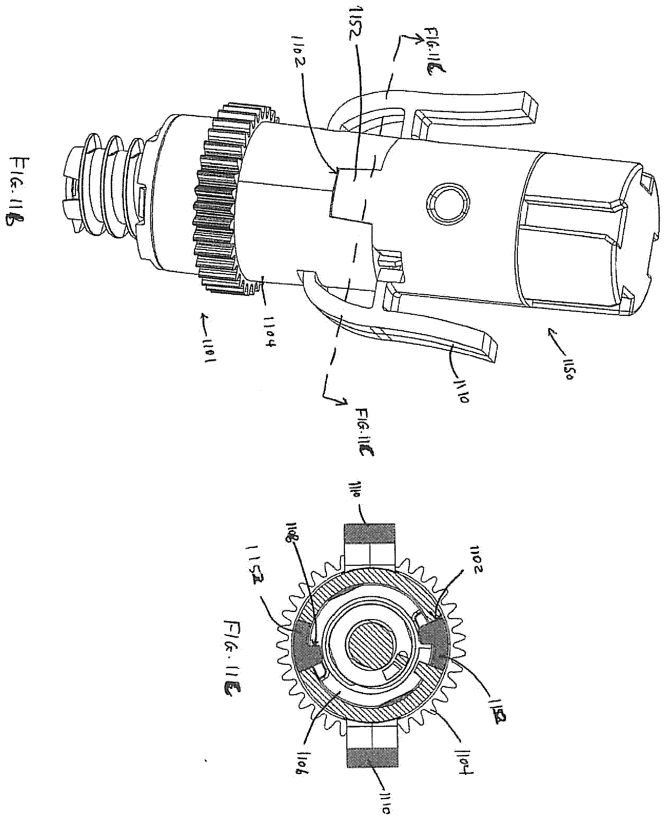

FIG. 11B is a perspective view of the assembled, closed alternative telescoping assembly 1101 of FIG. 11A, in accordance with an exemplary embodiment of the invention.

FIG. 11C is a cross-sectional view of the alternative telescoping assembly 1101 with assembly key 1150 of FIG. 11B, in accordance with an exemplary embodiment of the invention.

In some embodiments of the invention, the bit aperture 1102 of the cartridge gear 1104 aligns with a key slot 1108 of the pushing nut screw 1106, enabling the locking bit 1152 of the assembly key 1150 to be inserted through the bit aperture 1102 and into the key slot 1108 preventing movement of the cartridge gear 1104 relative to the pushing nut screw 1106. In an embodiment of the invention, prevention of movement between the cartridge gear 1104 relative to the pushing nut screw 1106 prevents rotation of the assembly 1101, thereby preventing unintended extension of the assembly 1101.

In an embodiment of the invention, assembly key 1150 has a pair of clips 1110 for removal of the assembly key 1150 from the telescoping assembly 1101. In an embodiment of the invention, the pair of clips 1110 are squeezed sufficiently to cause deformation of the assembly key 1150 and to release the assembly key 1150 from the bit aperture 1102 and the key slot 1108. The use of deformable clips 1110 may make removal of assembly key 1150 easier for a nervous end user who may be reluctant to use force to remove assembly key 150 and/or who may become nervous that he might have broken the telescoping assembly 101 when he feels a snap giving way upon removal of assembly key 150. In some embodiments, deformation is elastic. In some embodiments, deformation is plastic. In some embodiments, deformation is a combination of elastic and plastic.

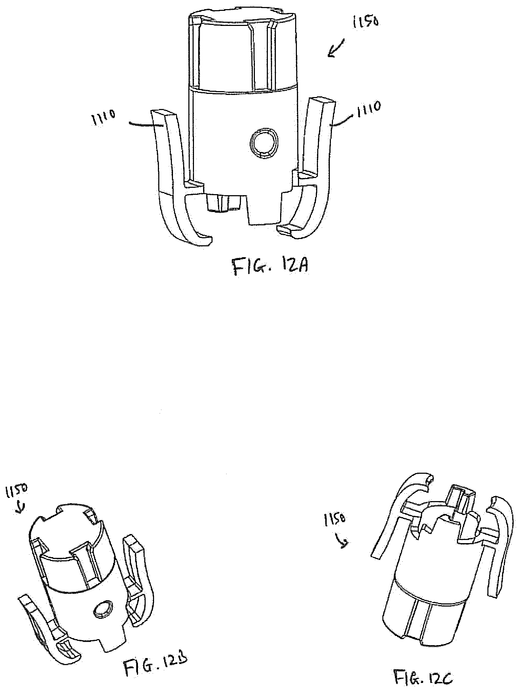

FIGS. 12A-12C are perspective views of the alternative assembly key 1150, in accordance with an exemplary embodiment of the invention.

In an embodiment of the invention, the assembly key 150, 1150 uses the same interface to attach to the telescoping assembly 101, 1101 as an assembly machine used for assembling the components of the telescoping assembly 101, 1101. In some embodiments of the invention, assembly key 150, 1150 is used as a linkage between the assembly machine and the telescoping assembly 101, 1101 during manufacturing. In an embodiment of the invention, after the telescoping assembly 101, 1101 is assembled, it is attached to a plunger of the drug delivery system, optionally by screwing. In an embodiment of the invention, the assembly key 150, 1150 provides a handle for manual or automatic screwing of the pushing nut cover into the plunger without extending, collapsing, screwing and/or unscrewing the telescoping assembly 150, 1150.

In an embodiment of the invention, the assembly key 150, 1150, used as a link between the telescoping assembly and the assembly machine during manufacturing, helps the assembly machine recognize the orientation of the telescoping assembly 101, 1101 during assembly, which can be important for proper assembly of the telescoping assembly 101, 1101 and insertion of it into the plunger. In an embodiment of the invention, mechanical sensing of the telescoping assembly's orientation using the assembly key obviates the need for more expensive optical sensing equipment.

In some embodiments of the invention, assembly keys as described herein are provided with a distinguishing feature, for example color, for easy identification by the customer for easier removal and/or disposal.

It is expected that during the life of a patent maturing from this application many relevant securing technologies will be developed and the scope of the terms securing is intended to include all such new technologies a priori.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.
As used herein, the singular form "a", "an" and "the" include plural references unless the context clearly dictates otherwise. For example, the term "a compound" or "at least one compound" may include a plurality of compounds, including mixtures thereof.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A method of securing an assembly of a drug delivery system, comprising the steps of:
threadingly interconnecting at least a first component (102) and a second component (104) of the assembly by a screw thread, thereby assembling a telescoping assembly (101) in a closed configuration thereof ready for placement into the drug delivery system, wherein relative rotation between said first and second components (102, 104) causes extension of said telescoping assembly (101);
inserting a locking bit (602) of an assembly key (150) through a bit aperture (502) of the first component (102) and into a slot of the second component (104), aligned with the bit aperture (502), thereby securing the telescoping assembly (101) in said closed configuration, preventing relative rotation between said first component (102) and said second component (104) and preventing linear extension of the telescoping assembly (101); and
transporting the assembly with the assembly key (150) installed.

2. The method according to claim 1, wherein the inserting step further comprises removably inserting at least one clip (604) of the assembly key (150) through a corresponding at least one counterpart clip aperture (504) of the first component (102) of the telescoping assembly (101) to removably attach the assembly key (150) to the telescoping assembly (101).

3. The method according to any of the preceding claims, wherein the first component (102) is a driver component (202) and the second component (104) is an actuating component (210).

4. The method according to any one of the preceding claims, wherein the assembly key (150) includes a handle on a proximal end thereof, and further comprising the step of screwing a pushing nut screw (204) and/or a pushing nut cover (210) of the telescoping assembly (101) into a plunger of the drug delivery system via the handle of the assembly key (150).

5. A system for preventing unintended linear extension of an assembly of a drug delivery system during transport, comprising:
a telescoping assembly (101) including at least a first component (102) and a second component (104) threadingly interconnected by a screw thread in a closed configuration thereof, wherein relative rotation between said first and second components (102, 104) causes extension of said assembly; and,
an assembly key (150) including a locking bit removably inserted through a bit aperture (502) of the first component (102) and into a slot of the second component (104), aligned with the bit aperture (502), securing the assembly in said closed configuration,
wherein the locking bit (602) of the assembly key (150) prevents rotation of at least the first component (102) and the second component (104) with respect to each other, thereby preventing linear extension of the closed telescoping assembly (101) during transport.

6. The system according to claim 5, wherein the first component (102) of the assembly includes an actuating component (210) for pushing a plunger of the drug delivery system.

7. The system according to claim 5 or 6, wherein the second component (104) includes a driver component (202).

8. The system according to any one of claims 5 - 7, wherein the assembly key (150) further comprises at least one clip (604) for removably attaching the assembly key (150) to a corresponding at least one counterpart clip aperture (504) of the telescoping assembly (101).

## Patentansprüche

1. Verfahren zum Sichern einer Anordnung eines Arzneimittelabgabesystems, umfassend die Schritte:
die mindestens eine erste Komponente (102) und eine zweite Komponente (104) der Anordnung durch ein Schraubengewinde gewindemäßig miteinander verbinden, wodurch eine Teleskopanordnung (101) in einer geschlossenen Konfiguration davon zusammengesetzt wird, die bereit ist, in das Arzneimittelabgabesystem eingesetzt zu werden, wobei eine relative Drehung zwischen der ersten und der zweiten Komponente (102, 104) eine Verlängerung der Teleskopanordnung (101) bewirkt;
einen Schließbart (602) eines Montageschlüssels (150) durch eine Bartöffnung (502) der ersten Komponente (102) und in einen Schlitz der zweiten Komponente (104) einführen, der mit der Bartöffnung (502) ausgerichtet ist, wodurch die Teleskopanordnung (101) in der geschlossenen Konfiguration gesichert wird, wodurch eine relative Drehung zwischen der ersten Komponente (102) und der zweiten Komponente (104) verhindert wird und eine lineare Verlängerung der Teleskopanordnung (101) verhindert wird; und die Anordnung mit dem installierten Montageschlüssel (150) transportieren.

2. Verfahren nach Anspruch 1, wobei der Einführungsschritt ferner das entfernbare Einführen mindestens einer Klemme (604) des Montageschlüssels (150) durch eine entsprechende mindestens eine Gegenstück-Klemmenöffnung (504) der ersten Komponente (102) der Teleskopanordnung (101) umfasst, um den Montageschlüssel (150) entfernbar an der Teleskopanordnung (101) zu befestigen.

3. Verfahren nach einem der vorhergehenden Ansprüche, wobei die erste Komponente (102) eine Treiberkomponente (202) und die zweite Komponente (104) eine Betätigungskomponente (210) ist.

4. Verfahren nach einem der vorhergehenden Ansprüche, wobei der Montageschlüssel (150) einen Handgriff an einem proximalen Ende davon aufweist und ferner den Schritt des Einschraubens einer Druckmutterschraube (204) und/oder einer Druckmutterabdeckung (210) der Teleskopanordnung (101) in einen Kolben des Arzneimittelabgabesystems über den Handgriff des Montageschlüssels (150) umfasst.

5. System zum Verhindern einer unbeabsichtigten linearen Ausdehnung einer Anordnung eines Arzneimittelabgabesystems während des Transports, umfassend:
eine Teleskopanordnung (101), die mindestens eine erste Komponente (102) und eine zweite Komponente (104) aufweist, die durch ein Schraubengewinde in einer geschlossenen Konfiguration davon gewindemäßig miteinander verbunden sind, wobei eine relative Drehung zwischen der ersten und der zweiten Komponente (102, 104) eine Verlängerung der Anordnung bewirkt; und,
einen Montageschlüssel (150), der einen Schließbart umfasst, der entfernbar durch eine Bartöffnung (502) der ersten Komponente (102) und in einen Schlitz der zweiten Komponente (104) eingeführt ist, die mit der Bartöffnung (502) ausgerichtet ist, was die Anordnung in der geschlossenen Konfiguration sichert,
wobei der Schließbart (602) des Montageschlüssels (150) eine Drehung von mindestens der ersten Komponente (102) und der zweiten Komponente (104) in Bezug zueinander verhindert, wodurch eine lineare Ausdehnung der geschlossenen Teleskopanordnung (101) während des Transports verhindert wird.

6. System nach Anspruch 5, wobei die erste Komponente (102) der Anordnung eine Betätigungskomponente (210) zum Drücken eines Kolbens des Arzneimittelabgabesystems umfasst.

7. System nach Anspruch 5 oder 6, wobei die zweite Komponente (104) eine Treiberkomponente (202) umfasst.

8. System nach einem der Ansprüche 5 bis 7, wobei der Montageschlüssel (150) ferner mindestens eine Klemme (604) zum lösbaren Befestigen des Montageschlüssels (150) an einer entsprechenden mindestens einen Gegenstück-Klemmenöffnung (504) der Teleskopanordnung (101) umfasst.

## Revendications

1. Procédé pour fixer un système d'administration de médicament, comprenant les étapes de :
relier entre eux, par des filetages, un premier composant (102) et un deuxième composant (104) du système par un filetage de vis, assembler par cela un système télescopique (101) dans une configuration fermée de celui-ci, prêt à être inséré dans un système d'administration de médicament, la rotation relative entre le premier et le deuxième composant (102, 104) entraînant une extension du système télescopique (101),
insérer un élément de verrouillage (602) d'une clé de système (150) au travers d'une ouverture d'élément (502) du premier composant (102) et dans une fente du deuxième composant (104), alignée avec l'ouverture d'élément (502), fixant par cela le système télescopique (101) dans ladite configuration fermée, évitant une rotation relative entre le premier composant (102) et le deuxième composant (104) et évitant une extension linéaire du système télescopique (101), et
transporter le système avec la clé de système (150) installée.

2. Procédé selon la revendication 1, **caractérisé en ce que** l'étape d'insertion comprend en outre insérer de manière amovible au moins une attache (604) de la clé de système (150) au travers d'au moins une ouverture de contre-attache (504) correspondante du premier composant (102) du système télescopique (101) pour fixer la clé de système (150) de manière amovible au système télescopique (101).

3. Procédé selon l'une des revendications précédentes, **caractérisé en ce que** le premier composant (102) est un composant d'entraînement (202) et que le deuxième composant (104) est un composant d'actionnement (210).

4. Procédé selon l'une des revendications précédentes, **caractérisé en ce que** la clé de système (150) comprend une poignée à l'extrémité proximale de celle-ci et **en ce que** le procédé comprend en outre l'étape de visser une vis d'écrou poussoir (204) et/ou un cache d'écrou poussoir (210) du système télescopique (101) dans un piston du système d'administration via la poignée de la clé de système (150).

5. Système pour éviter une extension linéaire non voulue d'un assemblage d'un système d'administration de médicament pendant le transport, comprenant
un système télescopique (101) comportant au moins un premier composant (102) et un deuxième composant (104) reliés entre eux, par des filetages, par un filetage de vis dans une configuration fermée de celui-ci, la rotation relative entre le premier et le deuxième composant (102, 104) entraînant une extension du système, et
une clé de système (150) comportant un élément de verrouillage inséré de manière amovible au travers d'une ouverture d'élément (502) du premier composant (102) et dans une fente du deuxième composant (104), alignée avec l'ouverture d'élément (502), fixant par cela le système télescopique (101) dans ladite configuration fermée,
**caractérisé en ce que** l'élément de verrouillage (602) de la clé de système (150) évite la rotation relative entre au moins le premier composant (102) et le deuxième composant (104) et évite par cela une extension linéaire du système télescopique (101) fermé pendant le transport.

6. Système selon la revendication 5, **caractérisé en ce que** le premier composant (102) du système comprend un composant d'actionnement (210) pour pousser un piston du système d'administration de médicament.

7. Système selon la revendication 5 ou 6, **caractérisé en ce que** le deuxième composant (104) comprend un composant d'entraînement (202).

8. Système selon l'une des revendications 5 à 7, **caractérisé en ce que** la clé de système (150) comprend en outre au moins une attache (604) pour attacher la clé de système (150) de manière amovible à au moins une ouverture de contre-attache (504) correspondante du système télescopique (101).
